# EUROPEAN PATENT APPLICATION

(11) **EP 3 158 958 A2**
(43) Date of publication of application: **26.04.2017**
(21) Application number: 16191582.2
(22) Date of filing: 29.09.2016
(51) Int. Cl.: A61B 17/70, A61B 17/86

(54) **BIASED ANGLE SCREWS**

(30) Priority: 29.09.2015 US 201514869090
(71) Applicant: Stryker European Holdings I, LLC, Kalamazoo, MI 49002 (US)
(72) Inventor: BUSH, Charles L. JR., WAYNE, 07470 (US)
(74) Representative: Regimbeau

(57) **Abstract**

Several different embodiment pedicle screws are disclosed. The designs allow for increased angulation between the screw portions and coupling elements, which may be particuarly useful for cervical vertebrae fusion procedures. Methods of utilizing the pedicle screws are disclosed herein.

## Description

### BACKGROUND OF THE INVENTION

Spinal fusion procedures involving pedicle screw fixation are common spinal procedures for addressing back and neck **pain** in patients. In a typical procedure, a surgeon will install pedicle screws into the pedicles of adjacent vertebrae (along one or multiple levels of the spine) and thereafter place a spinal rod in coupling elements of pedicle screws. Once in the desired position, the pedicle screws may be locked with a set screw or the like thereby fixing the adjacent vertebrae in position. Whether conducted in conjunction with interbody fusion or across single or multiple levels of the spine, the use of pedicle screws connected by fixation rods is an important treatment method employed by spinal surgeons.

The different levels of the spine (i.e., cervical, thoracic and lumbar) provide different anatomical considerations for pedicle screw procedures. For instance, where the thoracic and lumbar regions generally include larger vertebral bodies that allow for easier pedicle screw placement, the cervical region includes rather small vertebral bodies that require a more precise implantation procedure, as well as a need for an overall lower profile system. Although typical pedicle screws include a coupling element that is polyaxially movable with respect to an anchor portion, the relative size of the cervical region requires more extreme movements of the coupling element with respect to the anchoring portion in order to allow for proper spinal rod placement.

To address this need, biased angle screws have been developed that allow for greater movement/angulation of the coupling element with respect to the anchoring element in one or more directions. For instance, U.S. Patent Nos. 6,974,460; 8,506,600; and 8,870,930, the disclosures of which are hereby incorporated by reference herein, disclose, *inter alia*, a biased angle pedicle screw that includes a multi-bore coupling element allowing for greater angulation in a given direction.

Nonetheless other pedicle screw designs are desirable to, among other things, address structural considerations and the overall profile of the pedicle screws within a patient.

### BRIEF SUMMARY OF THE INVENTION

Various embodiment biased angle pedicle screws are disclosed in the present application. Each addresses the need for increased angulation, while at the same time maintaining structural stability. This results in strong constructs with low profiles.

A first aspect of the present invention is a pedicle screw having a screw portion including a post, a coupling element including an inner surface and a bushing including an aperture and an outer surface. The post is received within the aperture and the outer surface contacts the inner surface so that screw portion is moveable with respect to the coupling element.

In other embodiments according to the first aspect, the post may be tapered, as may the aperture. The inner and outer surfaces may be spherical. The coupling element may include a U-shaped slot for receiving a spinal rod. The coupling element may also include a threaded portion and a set screw may be provided for engaging the threaded portion and retaining the spinal rod within the U-shaped slot. The coupling element may also include an opening through which the screw portion extends. The coupling element may include an angled surface adjacent the opening allowing for greater movement of the screw portion with respect to the coupling element. The screw portion, coupling element and bushing may be constructed in situ.

Another aspect of the present invention is another pedicle screw having a screw portion including a post and a coupling element including a slot for receiving the post. The post is receivable within the slot when the coupling element is oriented in a first position with respect to the screw portion and retained within the slot when the coupling element is oriented in a second position with respect to the screw portion.

In other embodiments according to the second aspect, the first and second positions may be orthogonal. The post may include a flange and a neck, the flange received within the coupling element. The slot may be elongate allowing for greater movement of the screw portion with respect to the coupling element in one direction. The coupling element may include a U-shaped slot for receiving a spinal rod. The coupling element may include a threaded portion, and a set screw may be provided for engaging the threaded portion and retaining the spinal rod within the U-shaped slot. The post may contact the spinal rod. The screw portion may include a head having an angled upper surface, the post extending from the angled upper surface. The coupling element may include an angled lower surface in contact with the angled upper surface of the head.

### BRIEF DESCRIPTION OF THE DRAWINGS

These and other features, aspects and advantages of the present of the present invention will become understood with regard to the following description, appended claims, and accompanying drawings where:
Fig. 1 is a cross-sectional view of a pedicle screw in accordance with a first embodiment of the present invention.
Fig. 2A is a cross-sectional view of a pedicle screw in accordance with another embodiment of the present invention.
Fig. 2B is a cross-sectional view of the pedicle screw of Fig. 2A taken along line A-A.
Fig. 3 is a cross-sectional view of a pedicle screw in accordance with another embodiment of the present invention;
Fig. 4A is a cross-sectional view of a pedicle screw in accordance with another embodiment of the present invention.
Figs. 4B-C are cross-sectional views of a pedicle screw similar to that of Fig. 4A but in accordance with another embodiment of the present invention.
Fig. 5A is a cross-sectional view of a pedicle screw in accordance with another embodiment of the present invention.
Fig. 5B is a perspective view of the pedicle screw of Fig. 5A.
Fig. 5C is a cross-sectional view of a pedicle screw in accordance with another embodiment of the present invention.
Fig. 5D is a perspective view of a screw portion of the pedicle screw of Fig. 5C.
Fig. 6A is a cross-sectional view of a pedicle screw in accordance with another embodiment of the present invention.
Fig. 6B is a bottom view of an opening of the coupling element of the pedicle screw of Fig. 6A.
Fig. 7 is a cross-sectional view of a pedicle screw in accordance with another embodiment of the present invention.
Fig. 8A and 8B are cross-sectional views of a pedicle screw in accordance with another embodiment of the present invention.
Fig. 8C is a side view of a coupling element of the pedicle screw shown in Figs. 8A and 8B.
Fig. 8D is a front view of the coupling element shown in Fig. 8C.
Fig. 9 is a cross-sectional view of a pedicle screw in accordance with another embodiment of the present invention.
Fig. 10 is a cross-sectional view of a coupling element of a pedicle screw in accordance with another embodiment of the present invention.
Fig. 11A is a cross-sectional view of a pedicle screw in accordance with another embodiment of the present invention.
Fig. 11B is another view of the pedicle screw of Fig. 11A.

### DETAILED DESCRIPTION

When referring to the specific directions in the following discussion of certain surgical instruments, it should be understood that such directions are described with regard to the surgical instruments orientation and position during exemplary application of the human body. Thus, as used herein, the term "proximal" means close to the heart, and the term "distal" means more distant from the heart. The term "anterior" means toward the front of the body or the face, and the term "posterior" means toward the back of the body. The term "medial" means toward the midline of the body, and the term "lateral" means away from the midline of the body. Also, as used herein, the terms "about," "generally" and "substantially" are intended to mean that slight deviations from absolute are included within the scope of the terms so modified.

Following below is a discussion of several different embodiment biased angle pedicle screws in accordance with the present invention. In each case, the focus of the description will be on the interface between a coupling element and a screw portion. It is to be understood that other elements of the pedicle screws may be similar to known pedicle screw components, as would be recognized by one of ordinary skill in the art. For instance, any suitable thread portion of the shaft of the pedicle screw can be implemented in any of the below embodiments. Likewise, where the below focus is on the relationship between the coupling element and screw portion, a set screw or other fastener may also be provided to cooperate with the coupling element to capture a spinal rod therein.

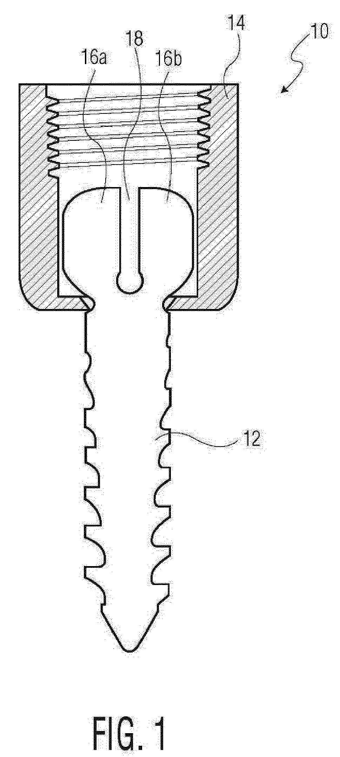

Fig. 1 depicts a first embodiment pedicle screw 10 which includes a screw portion 12 and a coupling element 14. Although coupling element 14 is substantially similar to a standard coupling element in existing pedicle screws, screw portion 12 includes a head 16 that is bifurcated into two portions 16a and 16b by a cut 18. The cut is made such that one of portions 16a or 16b is capable of flexing more than the other, thereby allowing the head to collapse more towards side than the other. This will in turn allow screw portion 12 to pivot/move with respect to coupling element 14 more in one direction(s).

Cut 18 may also be designed to allow for more pivoting of screw portion 12 with respect to coupling element 14, as opposed to in existing pedicle screw designs. In other words the ability of portions 16a and 16b to flex, even if one is not designed to flex more than the other, will generally allow for the additional pivoting. Cut 18 can be located in an offset location from the center of head 16, which acts to bias the direction of screw portion 12 within coupling element 14. This also may provide a degree of controlled contact between head 16 and coupling element 14, which may provide tactile feedback to a user and provisional positioning of the coupling element 14 when screw portion 12 is introduced into a bone. It is also contemplated to provide more than one cut in head 16 (e.g., a cut that extends orthogonal to cut 18). This type of design may allow for even more pivot, in other directions.

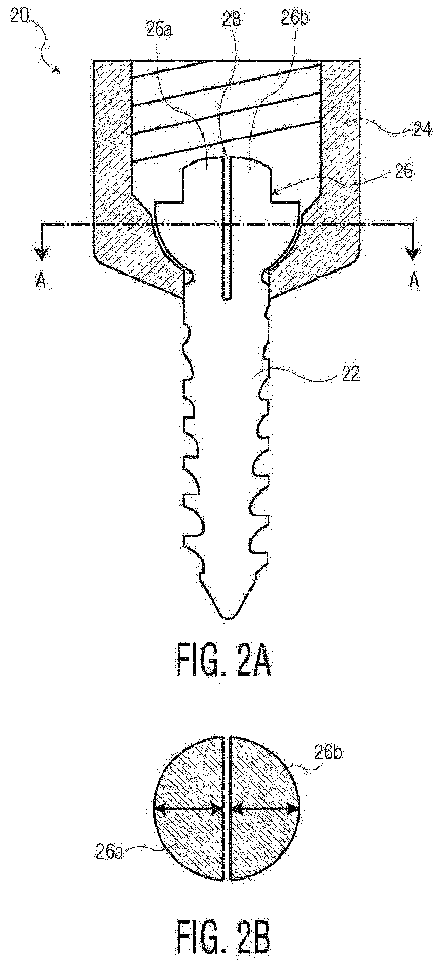

Figs. 2A and 2B depict another embodiment pedicle screw 20 including a screw portion 22 and a coupling element 24. Like in the design of pedicle screw 10, screw portion 22 includes a head 26 that is bifurcated into portions 26a and 26b by a cut 28. However, contrary to the female tool receiving aperture (not shown) of head 16, screw head 26 includes a male portion designed to couple with a female driving means. In addition, the design of head 26 is such that both increased angulation and drag is created between the screw 22 and coupling element 24. With regard to the latter, in existing pedicle screws, coupling elements and screw portions are generally allowed to freely move with respect to each other prior to rod placement and fixation, with nothing inhibiting the movement. This creates a "floppy" construction that can frustrate a surgeon during a spinal rod implantation. Both pedicle screws 10 and 20 may be designed to prevent this type of construction.

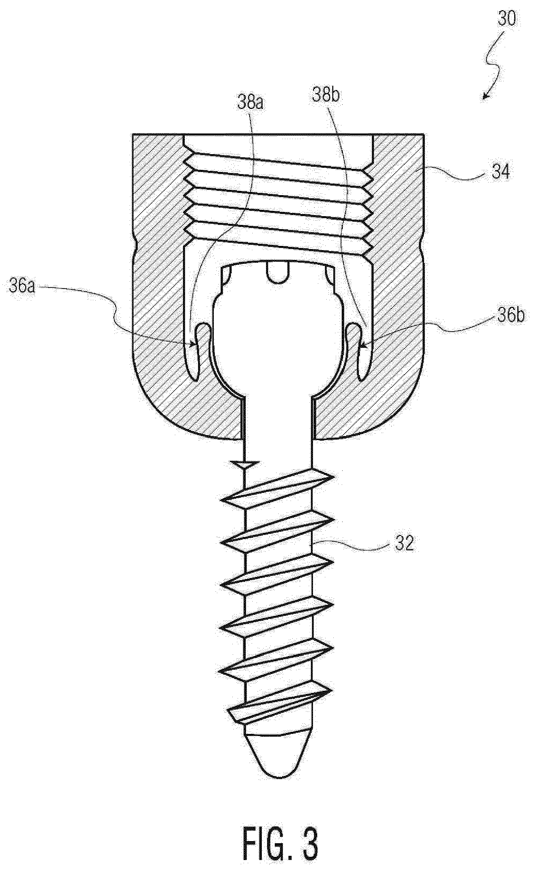

Fig. 3 depicts yet another embodiment pedicle screw 30, which includes a screw portion 32 and a coupling element 34. Screw 32 can be a standard screw like those utilized in existing pedicle screws. On the other hand, coupling element 34 includes arms 36a and 36b, which act to both capture screw 32 and allow for movement of the screw with respect to coupling element 34. In the design shown, cuts 38a and 38b define arms 36a and 38b, respectively, and allow them to flex. Because cut 38a is deeper within coupling element 34 than cut 38b, arm 36a is allowed to flex more than arm 36b. Thus, screw 32 is capable of moving with respect to coupling element 34 more in one direction that in the other. In addition, like in screws 10 and 20, arms 36a and 36b can provide drag on the screw thereby preventing a "floppy" construction.

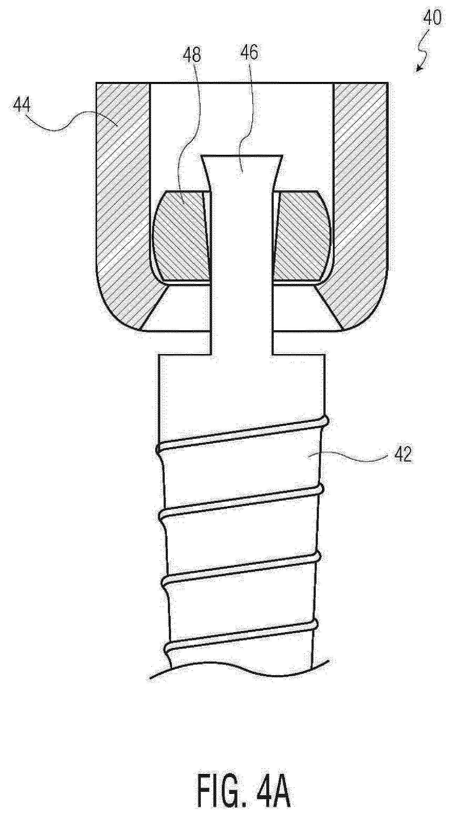

Fig. 4A depicts a pedicle screw 40 including a screw 42 and a coupling element 44. Screw 42 includes a post 46 in lieu of a standard screw head, and a bushing 48 is provided for receiving post 46. Coupling element 44 is designed to accept bushing 48 and allow for its movement therein. In this regard, bushing 48 includes a spherical outer surface that cooperates with a spherical inner surface of coupling element 44. This in turn allows for the polyaxial movement of screw 42 with respect to coupling element 44. It is contemplated to provide bushing with a tapered bore for receiving and fixedly retaining tapered post 46 therein. Because of bushing 48, coupling element 44 can include a wider lower opening than in existing pedicle screws, which allows for increased angulation between screw 42 and coupling element 44 to be achieved.

The design of pedicle screw 40 also allows for the coupling of screw 42 and coupling elements 44 at any time during a surgical procedure. In other words, where standard pedicle screws are typically provided with pre-coupled screw portions and coupling elements, pedicle screw 40 allows for easy attachment of coupling element 44 to screw 42 due to the cooperation between post 46 and bushing 48. This allows for a procedure in which screw 42 is implanted in the spine and coupling element 44 is thereafter be attached. Such a modular design allows for significant flexibility during a pedicle screw procedure.

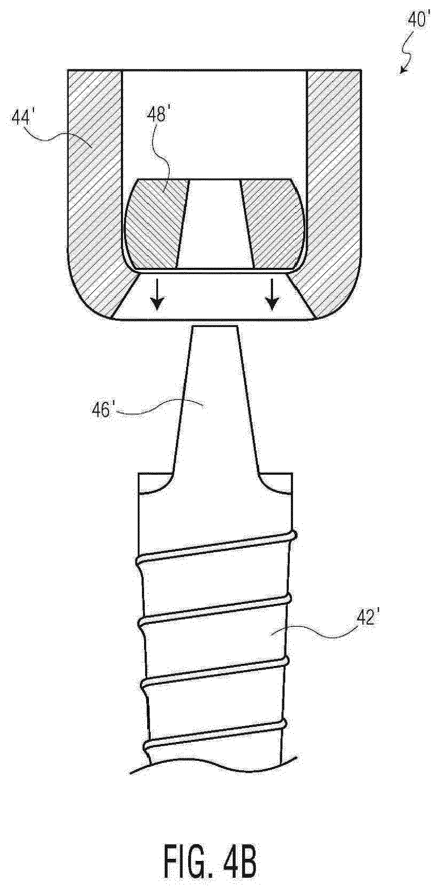

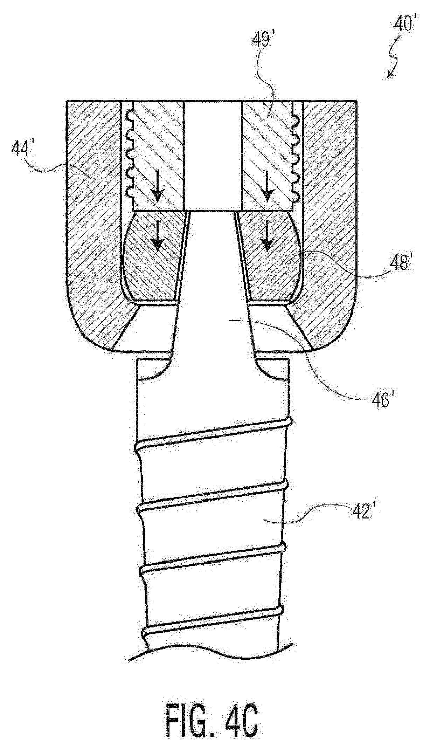

An alternative embodiment similar to pedicle screw 40 is shown in Figs. 4B-C. There, screw 40' includes a screw 42' and a coupling element 44'. The former includes a tapered post 46' that cooperates with a bushing 48' having a tapered opening. Upon application of a set screw or blocker 49' bushing 48' is moved further onto tapered post 46' to aid in the locking of the assembly. The final locked position is shown in Fig. 4C.

It is envisioned that any of the screw assemblies shown in Figs. 4A-C could be preassembled at a manufacturing site, or could be designed as modular screws to be assembled in situ. It is also envisioned to have posts 46 or 46' designed to protrude above bushing 48 or 48' so as to engage a set screw or rod. Additionally, it is contemplated to form the opening of bushing 48 or 48' offset to a midline of the component. This may create a nature bias or offset of the screw portions with respect to the coupling elements.

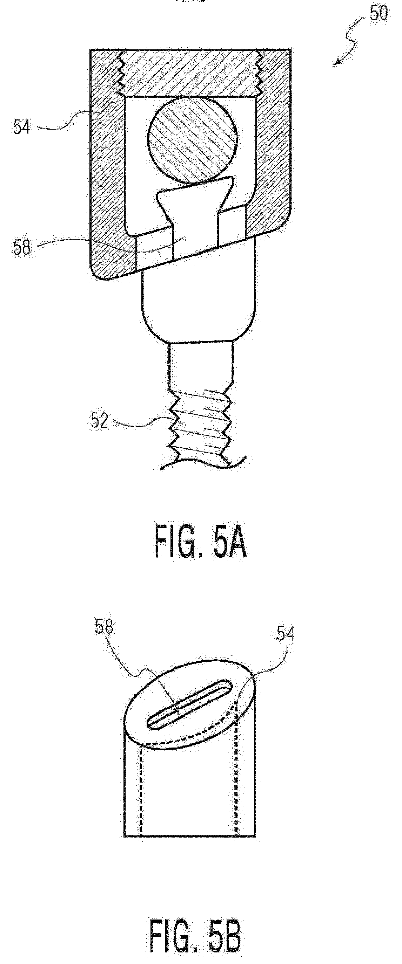

Figs. 5A and 5B depict yet another embodiment pedicle screw 50 including a screw portion 52 and a coupling element 54. As opposed to the typical screw head and coupling element association, screw 52 includes a capture post 58 and coupling element 54 includes a slot 58. This configuration allows for movement of screw 52 with respect to coupling element 54 only along the path of slot 58. In addition, this design allows for modularity similar to that described above in connection with pedicle screw 40. However, as opposed to the bushing design of pedicle screw 40, the design of pedicle screw 50 allows for capture post 56 to be inserted within 58 while the coupling element 54 is in a first orientation. Thereafter, coupling element 54 can be rotated to a second orientation to retain capture post 56 therebetween. For instance, it is contemplated to provide post 56 with an elongate design so that it can be received within slot 58 while coupling element 52 is in a first orientation, but retained upon a 90 degree rotation of the coupling element with respect to the screw.

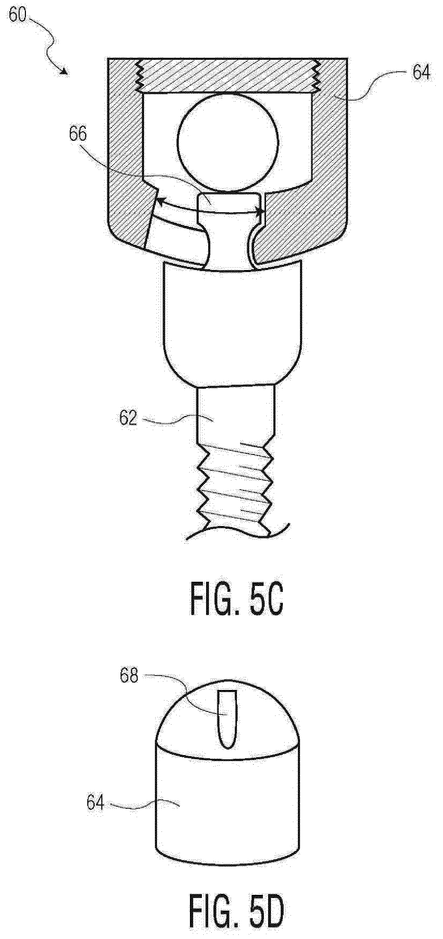

Figs 5C and 5D depict another embodiment pedicle screw 60 which is similar to the design of pedicle screw 50. In this regard, pedicle screw 60 includes a screw portion 62 having a capture post 66 and a coupling element 64 having a slot 68. However, as opposed to the asymmetrical design of coupling element 54, coupling element 64 includes a symmetrical design with a curved lower portion. Likewise, screw portion 62 includes a curved upper surface designed to cooperate with the lower surface of coupling element 64. This design allows for fluid movement of screw portion 62 with respect to coupling element 64 about several directions, whereas the design of screw 50 only allows for increased angulation in a single direction. Of course, the placement of slot 68 dictates the amount of movement in a given direction. As shown, slot 68 is only on one side of coupling element 64, thus only allowing increased angulation in that direction.

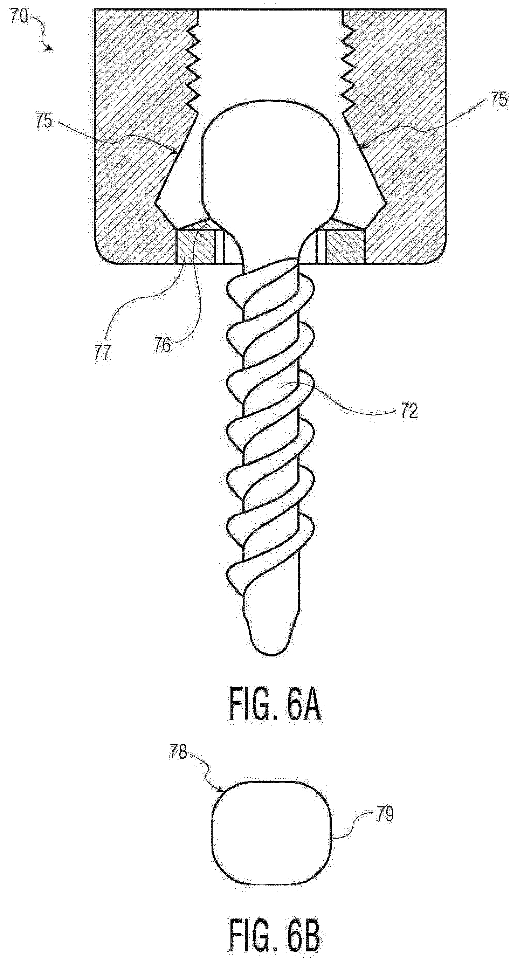

Figs 6A and 6B depict yet another embodiment pedicle screw 70, which includes a screw portion 72 and a coupling element 74. While screw portion 72 is of a typical screw design, coupling element 74 includes angled flats 75 within its interior, and a spring washer 76 and locking ring 77 construct at its lower portion. Although spring washer 76 and locking ring 77 are shown as separate elements, it is envisioned that such elements could be combined into a single component for use in pedicle screw 70. The spring washer 76 and locking ring 77 construct allows for a head of screw portion 74 to be retained within coupling element 74 and in certain embodiments the construct can provide a modular design like is discussed above in connection with certain other embodiments. In addition, and with reference to Fig. 6B, coupling element 74 is provided with an opening 78 at its lower portion that is of a rounded design, but includes flats 79. The flats are designed to allow for more movement in one direction as opposed to another, and the rounded corners of the design are aimed at providing fluid polyaxial movement in all directions. Opening 78 can also be oversized given the inclusion of the above-discussed spring washer 76 and locking ring 77 construct. This allows the screw portion 72 to move side to side to allow for additional angular positions.

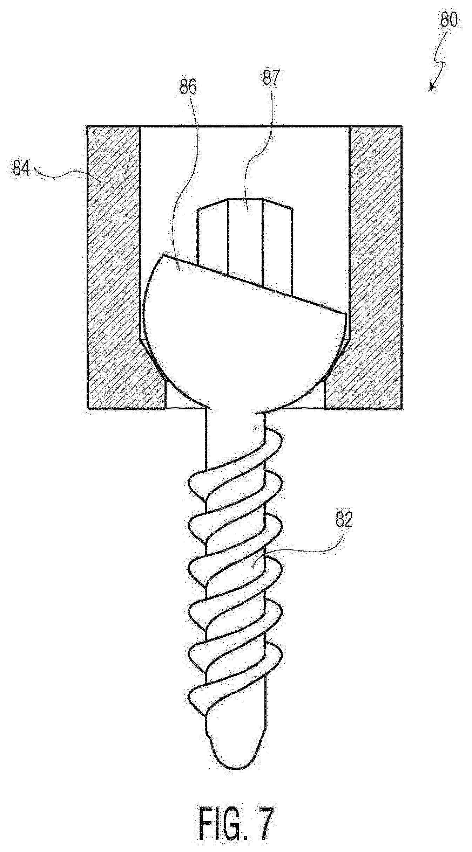

Fig. 7 depicts a pedicle screw 80 including a screw portion 82 and a coupling element 84. While coupling element 84 may be of any design, including like that of existing pedicle screws, screw 82 includes an angled head 86 having a male coupling portion 87 for coupling with a female driver or the like. The angled nature of head 86 allows the shank of the screw to be initially offset more in one direction than another, which in turn allows for greater overall angulation in that direction. Thus, the desired biased angularity can be achieved. The arrangement of coupling 87 with respect to the longitudinal axis of the screw (i.e., they are in parallel relationship), allows for screw 82 to be inserted into the pedicle in a normal fashion. This would not be achievable if the coupling 87 were angled like head 86. It is envisioned that a female coupling (not shown) that is also aligned with the longitudinal axis of the screw could alternatively be provided.

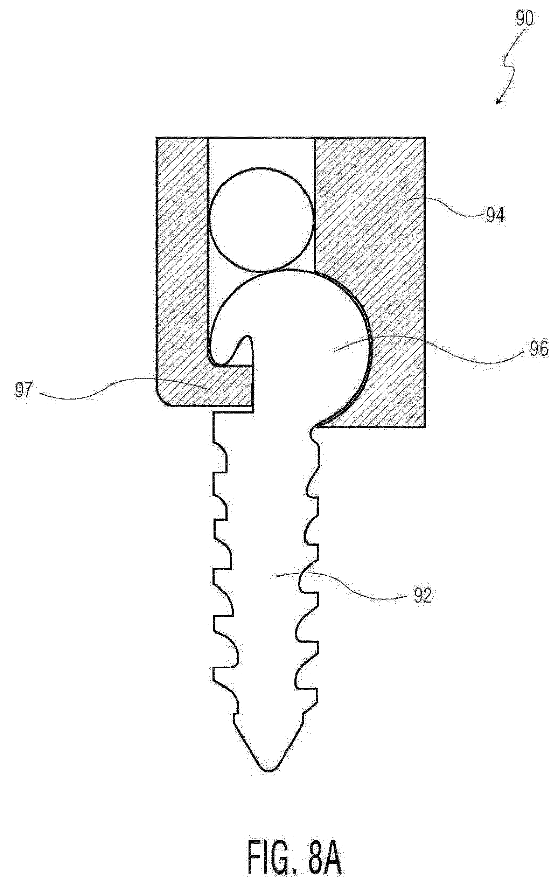

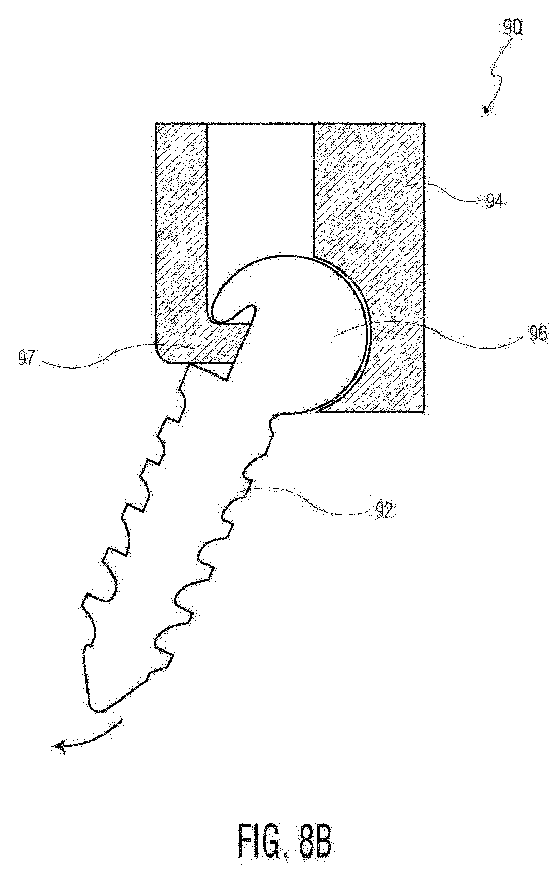

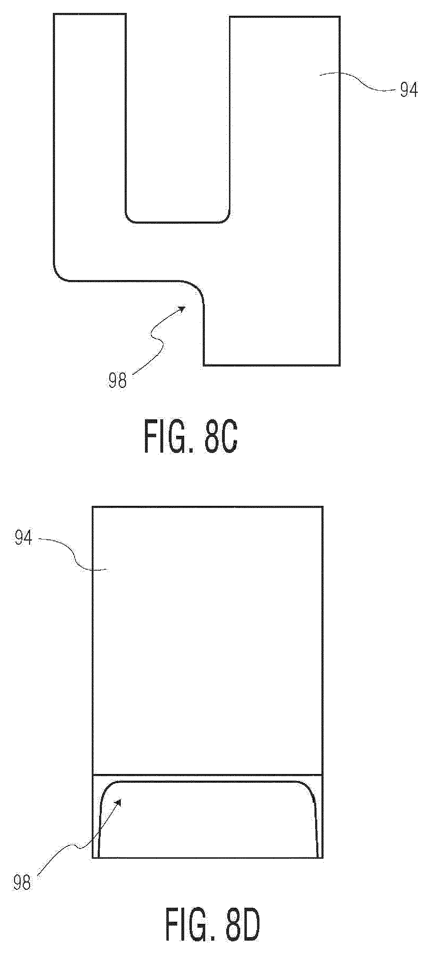

Figs. 8A through 8D depict yet another embodiment pedicle screw 90. Like the other pedicle screws discussed above, pedicle screw 90 includes a screw portion 92 and a coupling element 94. Screw portion 92 is provided with a head 96 that is shaped in a hook-like fashion, and coupling element 94 includes a projection 97 in its interior for engagement with hook-like head 96. In addition, as is best shown in Figs. 8C and 8D, coupling element 94 includes a cutout 98 at its lower portion on one side for allowing more angulation of the screw in that direction. Pedicle screw 90 is yet another modular-type design which allows for screw portion 92 to first be implanted in the pedicle of a patient and coupling element 94 to thereafter be attached, albeit with only two components. The hook-like design of head 96 allows for an assembly angle of near 90 degrees, which is far outside of the typical 50 degree working angles. This ensures that, once assembled, coupling element 94 remains attached to screw portion 92. In addition to the foregoing, coupling element 94 may include interior surfaces that cooperate with exterior surfaces of head 96 to allow for fluid polyaxial movement, again, more within one direction than in others. For instance, the remainders of head 96 and the interior of coupling element 94 are shown as being of cooperating spherical shapes.

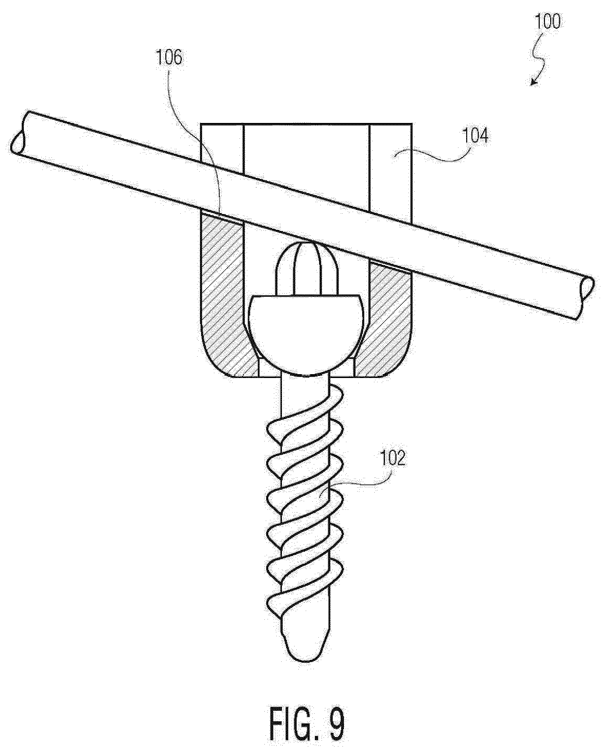

Fig. 9 depicts yet another embodiment and pedicle screw 100 including a screw portion 102 and a coupling element 104. Where screw portion 102 may be of a typical pedicle screw portion design, coupling element 104 includes an angulated rod receiving surface 106. In other words, where typical pedicle screws include a coupling element with a U-shaped rod receiving slot that is situated orthogonal to the longitudinal axis of the pedicle screw, pedicle screw 100 includes a surface 106 for receiving the rod that is situated at an angle other than 90 degrees with respect to its longitudinal axis. This design provides an initial offset of screw portion 92 with respect to a spinal rod received within coupling element 94. Therefore, even with a standard polyaxial relationship between screw portion 92 and coupling element 94, increased angulation is possible in one direction.

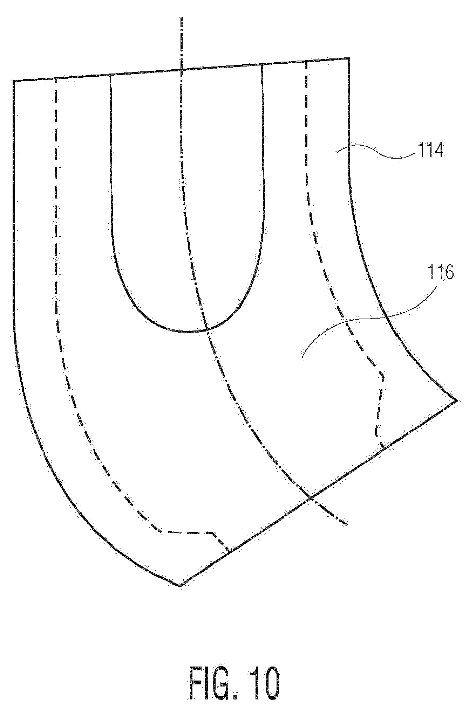

Fig. 10 depicts a coupling element 114 designed to be utilized with a standard screw portion, like many of those described above. As opposed to existing pedicle screw designs which include a coupling element having a single straight bore, or multiple bores oriented transverse to one another, coupling element 114 includes a curved bore 116. This design acts to achieve the desired biased angulation by initially offsetting the screw with respect to an implanted spinal rod. For instance, curved bore 116 can be configured such that a screw portion is initial set to a 40 degree angle in a neutral position. Further polyaxial movement results in a significant additional angulation in a given direction. For example, with the 40 degree neutral position and a 20 degree polyaxial motion, a bias of 60 degrees in one direction can be achieved.

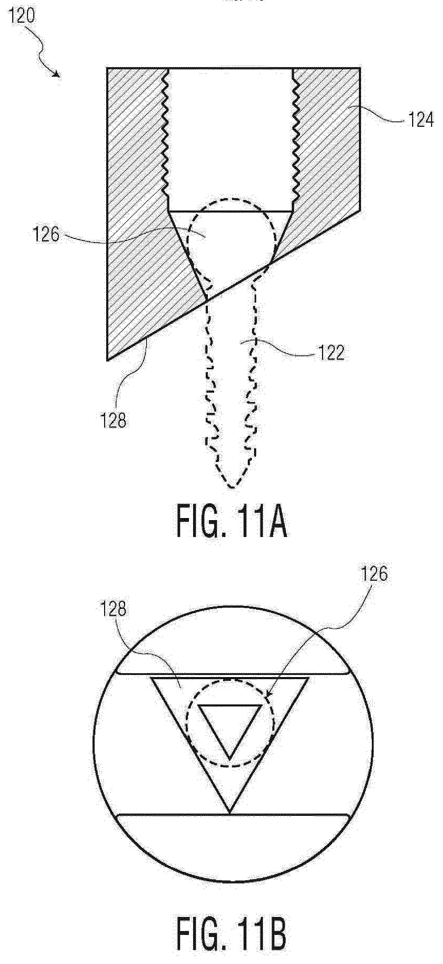

Figs. 11A and 11B depict a final illustrated embodiment pedicle screw 120 in accordance with the present invention. Screw 120 includes a screw portion 122 and a coupling element 124. Screw portion 122 includes a rounded head 126 while coupling element 124 includes a angled lower surface 128. In addition, the coupling element includes a triangular shaped opening 128 through which screw 120 extends. In fact, the shaft of the screw is capable of angulating into the corners of opening 128, thereby providing additional agulation in a given direction.

Any of the foregoing designs may be employed in spinal fusion procedures in accordance with standard uses of pedicle screws. Such screws may or may not be utilized in connection with interbody devices or the like. As noted above, the increased angulation provided by the designs according to the present invention are particularly useful in cervical applications, although others uses are clearly contemplated. Additionally, the above-disclosed screws that allow for modularity allow for in situ coupling of screw portions and coupling elements. Thus, the screw portions may first be implanted and the coupling elements thereafter introduced. This may provide additional benefits over typical pedicle screws.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A pedicle screw comprising:
a screw portion including a post;
a coupling element including an inner surface; and
a bushing including an aperture and an outer surface,
wherein the post is received within the aperture and the outer surface contacts the inner surface so that screw portion is moveable with respect to the coupling element.

2. The pedicle screw of claim 1, wherein the post is tapered.

3. The pedicle screw of claim 2, wherein the aperture is tapered.

4. The pedicle screw of claim 1, wherein the inner and outer surfaces are spherical.

5. The pedicle screw of claim 1, wherein the coupling element includes an opening through which the screw portion extends.

6. The pedicle screw of claim 5, wherein the coupling element includes an angled surface adjacent the opening allowing for greater movement of the screw portion with respect to the coupling element.

7. The pedicle screw of claim 1, wherein the screw portion, coupling element and bushing are constructed in situ.

8. A pedicle screw comprising:
a screw portion including a post; and
a coupling element including a slot for receiving the post,
wherein the post is receivable within the slot when the coupling element is oriented in a first position with respect to the screw portion and retained within the slot when the coupling element is oriented in a second position with respect to the screw portion.

9. The pedicle screw of claim 8, wherein the first and second positions are orthogonal.

10. The pedicle screw of claim 8, wherein the post includes a flange and a neck, the flange received within the coupling element.

11. The pedicle screw of claim 8, wherein the slot is elongate allowing for greater movement of the screw portion with respect to the coupling element in one direction.

12. The pedicle screw of claim 8, wherein the coupling element includes a threaded portion.

13. The pedicle screw of claim 12, further including a set screw for engaging the threaded portion and retaining the spinal rod within the U-shaped slot and wherein the post contacts the spinal rod.

14. The pedicle screw of claim 8, wherein the screw portion includes a head having an angled upper surface, the post extending from the angled upper surface.

15. The pedicle screw of claim 14, wherein the coupling element includes an angled lower surface in contact with the angled upper surface of the head.
